# EUROPEAN PATENT APPLICATION

(11) **EP 4 119 655 A1**
(43) Date of publication of application: **18.01.2023**
(21) Application number: 21768441.4
(22) Date of filing: 05.03.2021
(51) Int. Cl.: C12N 1/16, C12N 9/10, A61K 8/9728, A23K 10/16, A23L 33/14, A61K 36/06, C12P 7/64, C12R 1/645

(54) **YARROWIA SP. VARIANT AND METHOD FOR PREPARING FAT BY USING SAME**

(30) Priority: 09.03.2020 KR 20200029137
(71) Applicant: CJ Cheiljedang Corporation, Seoul 04560 (KR)
(72) Inventor: JANG, Jiryang, Seoul 04560 (KR); LEE, Peter, Seoul 04560 (KR); BAE, Jee Yeon, Seoul 04560 (KR); KIM, Ju-yeon, Seoul 04560 (KR); PARK, Hye Min, Seoul 04560 (KR); KIM, Hyung Joon, Seoul 04560 (KR); PARK, Sang Min, Seoul 04560 (KR)
(74) Representative: Regimbeau
(86) International application number: PCT/KR2021/002774
(87) International publication number: WO 2021/182808

(57) **Abstract**

Provided is a variant strain of the genus *Yarrowia.* More specifically, provided are a variant strain of the genus *Yarrowia,* in which activity of phosphatidylethanolamine *N*-methyltransferase (PEMT) or phospholipid methyltransferase is inactivated, and a method of increasing a fat in the strain, including culturing the strain, or a method of preparing a fat.

## Description

### [Technical Field]

The present disclosure relates to yeast improvement for enhancement of fat content, and a preparation method thereof, and in particular, a variant strain of the genus *Yarrowia,* in which activity of phosphatidylethanolamine N-methyltransferase (PEMT) or phospholipid methyltransferase is inactivated, and thus a fat content is enhanced, and a method of increasing a fat in the strain by culturing the variant strain of the genus *Yarrowia* in a medium containing choline.

### [Background Art]

Oleaginous yeasts that produce fatty acid derivatives such as free fatty acids, fatty alcohols, fatty acid ethyl esters, alkanes, *etc.* have received attention in various fields such as energy, cosmetics, foods, feed, *etc.,* and various studies related thereto have been conducted. Among them, triacylglycerol (TAG), which is a precursor of fatty acid derivatives and is a fatty form available for long-term storage, has been studied for the synthesis of large quantities thereof.

As a method of improving TAG contents in oleaginous yeasts, strategies for enhancing TAG biosynthetic pathways (*e.g*., dga1-2, slc1, acl1-2, acc1, sct1, lro1), inhibiting TAG degradation (*e.g*., tgl1-4, faa1, pxa1-2), and inhibiting *beta-*oxidation (*e.g*., pox1-6, mfe1, pex1-32, pot1) are mainly used by many researchers (U.S. Patent Application Publication No. 2013-0344548 A1).

In recent years, various strategies have been attempted to improve the fat content in yeasts, but there has been no remarkable increase in the fat content. Accordingly, there is a demand for an alternative to improve the level of fat production while preventing deterioration of yeast growth.

### [Disclosure]

### [Technical Problem]

The present inventors found that the fat production may be increased by a variant strain, in which activity of phosphatidylethanolamine *N*-methyltransferase or phospholipid methyltransferase is inactivated, thereby completing the present disclosure.

### [Technical Solution]

An object of the present disclosure is to provide a variant strain of the genus *Yarrowia,* in which activity of phosphatidylethanolamine *N-*methyltransferase (PEMT) or phospholipid methyltransferase is inactivated.

Another object of the present disclosure is to provide a cosmetic composition, a food composition, a feed composition, or a medical composition, each including at least one of the variant strain of the genus *Yarrowia,* a culture of the strain, an extract of the strain, a dry product of the strain, a lysate of the strain, and a fat recovered from at least one of the strain, the culture, the extract, the dry product, and the lysate.

Still another object of the present disclosure is to provide a method of increasing a fat in a strain, including: culturing the variant strain of the genus *Yarrowia.*

Still another object of the present disclosure is to provide a method of preparing a fat, the method including: culturing the variant strain of the genus *Yarrowia.*

Still another object of the present disclosure is to provide use of the variant strain of the genus *Yarrowia,* the culture of the strain, the extract of the strain, the dry product of the strain, the lysate of the strain, the cosmetic composition, the food composition, the feed composition, the medical composition, or the culture of the variant strain of the genus *Yarrowia* in fat production.

### [Advantageous Effects]

The variant strain of the genus *Yarrowia* of the present disclosure may increase intracellular fat production by inactivation of activity of phosphatidylethanolamine *N*-methyltransferase (PEMT) or phospholipid methyltransferase.

A method of increasing a fat in the strain and a method of preparing a fat of the present disclosure can effectively increase fat production while preventing deterioration of growth of the strain at the same time.

A cosmetic composition, a food composition, a feed composition, and a medical composition of the present disclosure can provide improved functionality by increasing a fat content.

### [Best Mode]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

To achieve the above objects, one aspect of the present disclosure provides a variant strain of the genus *Yarrowia,* in which activity of phosphatidylethanolamine *N*-methyltransferase or phospholipid methyltransferase is inactivated.

As used herein, the "phosphatidylethanolamine *N*-methyltransferase (PEMT)" is a transferase enzyme that converts phosphatidylethanolamine (PE) to phosphatidylcholine (PC). Phosphatidylcholine produced via PEMT may be utilized in choline synthesis, membrane structure, and VLDL secretion to play a wide range of physiological roles. In the present disclosure, the phosphatidylethanolamine *N*-methyltransferase may be used interchangeably with PEMT. In the present disclosure, the phosphatidylethanolamine N-methyltransferase may be encoded by *cho2* gene.

As used herein, the "phospholipid methyltransferase" is an enzyme that catalyzes a methylation reaction of phosphatidylethanolamine (PE), and may play a role in synthesizing phosphatidylcholine (PC). In the present disclosure, the phospholipid methyltransferase may be encoded by *opi3* gene.

The inactivation of the activity of the phosphatidylethanolamine N-methyltransferase or phospholipid methyltransferase of the present disclosure means inactivation of activity of one of the two enzymes or inactivation of a combination thereof.

With respect to the objects of the present disclosure, the variant strain of the genus *Yarrowia,* in which activity of one of the two enzymes is inactivated or activities of both of them are inactivated, may have the increased fat content in the strain, as compared with the wild-type, but is not limited thereto.

Sequences of the "phosphatidylethanolamine *N*-methyltransferase" or "phospholipid methyltransferase" may be obtained from a public database, and examples of the public database may include GenBank of the NCBI, *etc.* For example, it may be phosphatidylethanolamine *N*-methyltransferase and/or phospholipid methyltransferase derived from the genus *Yarrowia* (*Yarrowia* sp.), and specifically, the phosphatidylethanolamine *N*-methyltransferase may include an amino acid sequence of SEQ ID NO: 2, and the phospholipid methyltransferase may include an amino acid sequence of SEQ ID NO: 12, but is not limited thereto. The polypeptide including the amino acid sequence of SEQ ID NO: 2 may be used interchangeably with a polypeptide having the amino acid sequence of SEQ ID NO: 2 or a polypeptide consisting of the amino acid sequence of SEQ ID NO: 2, and the polypeptide including the amino acid sequence of SEQ ID NO: 12 may be used interchangeably with a polypeptide having the amino acid sequence of SEQ ID NO: 12 or a polypeptide consisting of the amino acid sequence of SEQ ID NO: 12. Further, any polypeptide sequence may also be included, as long as it has the same activity as the above amino acid sequence.

Further, the polypeptide may include the amino acid sequence of SEQ ID NO: 2 of the phosphatidylethanolamine *N*-methyltransferase and/or the amino acid sequence of SEQ ID NO: 12 of the phospholipid methyltransferase, or an amino acid sequence having 30% or more homology or identity thereto, but is not limited thereto. Specifically, the polypeptide may include an amino acid sequence having at least 30%, 60%, 80%, 85%, 90%, 95%, 96%, 97%, 98%, or 99% or more homology or identity to SEQ ID NO: 2 of the phosphatidylethanolamine *N*-methyltransferase and/or SEQ ID NO: 12 of the phospholipid methyltransferase. Additionally, it is obvious that any polypeptide having an amino acid sequence with deletion, modification, substitution, or addition in part of the sequence may also be included within the scope of the present disclosure, as long as the amino acid sequence has homology or identity described above and exhibits efficacy corresponding to that of the polypeptide.

In other words, although described as "a protein or polypeptide consisting of an amino acid sequence of a specific SEQ ID NO" in the present disclosure, it is obvious that any polypeptide having an amino acid sequence with deletion, modification, substitution, or addition in part of the sequence may also be used in the present disclosure, as long as the polypeptide may have an activity identical or corresponding to that of a polypeptide consisting of the amino acid sequence of the corresponding SEQ ID NO. For example, it is obvious that any polypeptide may belong to the "polypeptide consisting of the amino acid sequence of SEQ ID NO: 2", as long as it has an activity identical or corresponding to that of the "polypeptide consisting of the amino acid sequence of SEQ ID NO: 2".

In the present disclosure, genes encoding the phosphatidylethanolamine *N*-methyltransferase and the phospholipid methyltransferase may be *cho2* and *opi3* genes, respectively. Polynucleotide sequences encoding the amino acids of the present disclosure may be polynucleotide sequence(s) of SEQ ID NO: 1 and/or SEQ ID NO: 11 encoding the phosphatidylethanolamine *N-*methyltransferase and/or the phospholipid methyltransferase, but are not limited thereto. Further, the polynucleotide sequences may include any sequence without limitation, as long as it has the same activity as that of the polynucleotide sequence. The *cho2* gene may be a nucleotide sequence encoding the amino acid sequence of SEQ ID NO: 2, and more specifically, may be a sequence including the nucleotide sequence of SEQ ID NO: 1, but is not limited thereto. The sequence including the nucleotide sequence of SEQ ID NO: 1 and the polynucleotide including the nucleotide sequence of SEQ ID NO: 1 may be used interchangeably with a polynucleotide having the nucleotide sequence of SEQ ID NO: 1, a polynucleotide consisting of the nucleotide sequence of SEQ ID NO: 1, and a gene consisting of the polynucleotide sequence of SEQ ID NO: 1.

The *opi3* gene may be a nucleotide sequence encoding the amino acid sequence of SEQ ID NO: 12, and more specifically, may be a sequence including a nucleotide sequence of SEQ ID NO: 11, but is not limited thereto. The sequence including the nucleotide sequence of SEQ ID NO: 11 and the polynucleotide including the nucleotide sequence of SEQ ID NO: 11 may be used interchangeably with a polynucleotide having the nucleotide sequence of SEQ ID NO: 11, a polynucleotide consisting of the nucleotide sequence of SEQ ID NO: 11, and a gene consisting of the polynucleotide sequence of SEQ ID NO: 11.

As used herein, the term "polynucleotide", which refers to a long-chain polymer of nucleotides formed by linking nucleotide monomers via covalent bonds, has a meaning collectively including DNA or RNA molecules. Nucleotides, which are the basic structural units of polynucleotides, include not only natural nucleotides but also modified analogs thereof in which sugar or base moieties are modified (see Scheit, Nucleotide Analogs, John Wiley, New York (1980); Uhlman and Peyman, Chemical Reviews, 90:543-584 (1990)).

The polynucleotide may include a polynucleotide encoding the phosphatidylethanolamine *N*-methyltransferase and/or the phospholipid methyltransferase polypeptide of the present disclosure or a polynucleotide sequence having at least 30%, 60%, 80%, 85%, 90%, 95%, 96%, 97%, 98%, or 99% or more homology or identity to the phosphatidylethanolamine N-methyltransferase and/or phospholipid methyltransferase polypeptide of the present disclosure. Additionally, it is obvious that any polynucleotide sequence with deletion, modification, substitution, or addition in part of the sequence may also be included within the scope of the present disclosure, as long as the polynucleotide sequence has this homology or identity and encodes the polypeptide.

Additionally, it is apparent that due to codon degeneracy, a polynucleotide which may be translated into a polypeptide including the amino acid sequence of SEQ ID NOs: 2 and/or 12 or a polypeptide including an amino acid sequence having 30% or more identity to SEQ ID NOs: 2 and/or 12 or a polypeptide having homology or identity thereto may also be included. Alternatively, a probe which may be prepared from a known gene sequence, for example, any polynucleotide sequence which hybridizes with a sequence complementary to all or a part of the polynucleotide sequence under stringent conditions to encode a polypeptide including an amino acid sequence having 30% or more identity to the amino acid sequence of SEQ ID NO: 2, may be included without limitation. The "stringent conditions" refer to the conditions which allow the specific hybridization between polynucleotides. Such conditions are specifically disclosed in the literature (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York). For example, the stringent conditions may include conditions under which genes having high homology or identity, genes having 30% or more, 70% or more, 80% or more, specifically 85% or more, specifically 90% or more, more specifically 95% or more, much more specifically 97% or more, particularly specifically 99% or more homology or identity hybridize with each other, while genes having homology or identity lower than the above homology or identity do not hybridize with each other; or may include ordinary washing conditions of Southern hybridization, i.e., washing once, specifically twice or three times, at a salt concentration and a temperature corresponding to 60°C, 1XSSC, 0.1% SDS, specifically 60°C, 0.1XSSC, 0.1% SDS, and more specifically 68°C, 0.1XSSC, 0.1% SSD.

Hybridization requires that two polynucleotides have complementary sequences, although mismatches between bases are possible depending on stringency of the hybridization. The term "complementary" is used to describe a relationship between nucleotide bases that may hybridize with each other. For example, with respect to DNA, adenosine is complementary to thymine, and cytosine is complementary to guanine. Therefore, the present disclosure may also include an isolated polynucleotide fragment complementary to the entire sequence as well as a polynucleotide sequence substantially similar thereto.

Specifically, a polynucleotide having homology or identity may be detected using hybridization conditions including a hybridization step at a Tₘ value of 55°C under the above-described conditions. Additionally, the Tₘ value may be 60°C, 63°C, or 65°C, but is not limited thereto, and may be appropriately controlled by those skilled in the art depending on the purpose thereof.

The appropriate stringency for hybridizing polynucleotides depends on the length and degree of complementarity of the polynucleotides, and these variables are well known in the art (e.g., J. Sambrook *et al.*, *supra*).

As used herein, the term "homology" or "identity" refers to the degree of relevance between two given amino acid sequences or nucleotide sequences, and may be expressed as a percentage. The terms "homology" and "identity" are often used interchangeably with each other.

The sequence homology or identity of conserved polynucleotides or polypeptides may be determined by standard alignment algorithms and may be used together with a default gap penalty established by the program being used. Substantially, homologous or identical sequences are generally expected to hybridize to all or at least about 50%, about 60%, about 70%, about 80%, or about 90% or more of the entire length of the sequences under moderate or highly stringent conditions. Polynucleotides that contain degenerate codons instead of codons in the hybridizing polynucleotides are also considered.

Whether any two polynucleotide or polypeptide sequences have homology, similarity, or identity may be determined by a known computer algorithm such as the "FASTA" program using default parameters as in Pearson et al. (1988) Proc. Natl. Acad. Sci. USA 85:2444. Alternatively, it may be determined by the Needleman-Wunsch algorithm (Needleman and Wunsch, 1970, J. Mol. Biol. 48:443-453), which is performed using the Needleman program of the EMBOSS package ("EMBOSS: The European Molecular Biology Open Software Suite", Rice et al., 2000, Trends Genet. 16:276-277) (version 5.0.0 or later) (GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994; and CARILLO et al. (1988) SIAM J Applied Math 48:1073). For example, homology, similarity, or identity may be determined using BLAST or ClustalW of the National Center for Biotechnology Information.

The homology, similarity, or identity of polynucleotides or polypeptides may be determined by comparing sequence information using, for example, the GAP computer program, such as Needleman et al. (1970), J Mol Biol. 48:443, as disclosed in Smith and Waterman, Adv. Appl. Math (1981) 2:482. In summary, the GAP program defines it as the value obtained by dividing the number of similarly aligned symbols (*i.e.,* nucleotides or amino acids) by the total number of the symbols in the shorter of the two sequences. Default parameters for the GAP program may include (1) a unary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl. Acids Res. 14:6745, as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for end gaps. Accordingly, as used herein, the term "homology" or "identity" refers to the relatedness between sequences.

The "inactivation" of the activity of the polypeptide or protein of the present disclosure refers to a case where the polypeptide or protein is not in any way expressed or a case where the polypeptide or protein is expressed but exhibits no activity or reduced activity, as compared with a natural wild-type strain, a parent strain, or a strain in which the corresponding polypeptide or protein is not modified. The inactivation or attenuation may be used interchangeably with the terms "attenuation", "down-regulation", "decrease", "reduce", *etc.* In this regard, the inactivation is a concept that includes a case where the activity of a polypeptide itself is reduced or eliminated, as compared with the activity of the polypeptide originally possessed by a microorganism, due to a modification in the gene encoding the polypeptide, a modification in an expression regulatory sequence, deletion of a part or the entirety of the gene, *etc.*; a case where the level of overall polypeptide activity within a cell is reduced, as compared with the wild-type strain or the strain before modification, due to inhibition of expression of the gene encoding the polypeptide, or inhibition of translation, *etc.;* a case where the gene is not in any way expressed; or a case where the gene is expressed but exhibits no activity; and a combination thereof.

In the present disclosure, the inactivation is not limited thereto, and may be achieved by applying various methods well known in the art (Nakashima N. et al., "Bacterial cellular engineering by genome editing and gene silencing". Int J Mol Sci. 2014; 15(2):2773-2793, Sambrook et al. Molecular Cloning 2012, *etc.).* Examples of the methods include a method of deleting a part or the entirety of a gene encoding the polypeptide on a chromosome; a method of replacing the gene encoding the polypeptide on the chromosome with a gene that is modified to reduce the activity of the enzyme; a method of introducing a modification into an expression regulatory sequence of the gene encoding the polypeptide on the chromosome; a method of replacing the expression regulatory sequence of the gene encoding the polypeptide with a sequence having a weak activity or no activity (e.g., a method of replacing a promoter of the gene with a promoter weaker than an endogenous promoter); a method of deleting a part or the entirety of the gene encoding the polypeptide on the chromosome; a method of introducing an antisense oligonucleotide (e.g., antisense RNA), which inhibits translation from an mRNA into the protein or polypeptide via complementary binding to a transcript of the gene encoding the polypeptide on the chromosome; a method of making the attachment of a ribosome impossible by forming a secondary structure by artificially adding a complementary sequence to the SD sequence on the front end of the SD sequence of the gene encoding the polypeptide; and a reverse transcription engineering (RTE) method, which adds a promoter for reverse transcription to the 3' terminal of the open reading frame (ORF) of the polynucleotide sequence of the gene encoding the polypeptide; or a combination thereof, but are not particularly limited thereto.

Specifically, the method of deleting a part or the entirety of the gene encoding the protein or polypeptide may be performed by replacing the polynucleotide encoding the endogenous target protein within the chromosome with a polynucleotide or a marker gene having a partially deleted nucleic acid sequence via a vector for chromosomal insertion into microorganisms. For example, a method of deleting a gene by homologous recombination may be used, but the method is not limited thereto. Further, the term "part", as used herein, may specifically refer to 1 to 300 nucleotides, more specifically 1 to 100 nucleotides, and even more specifically 1 to 50 nucleotides, although it may vary depending on the kinds of polynucleotide, and this may be appropriately decided by those skilled in the art. However, the part is not particularly limited thereto.

The method of deleting a part or the entirety of the gene may be performed by inducing a mutation using light such as ultraviolet rays or chemical substances, and selecting a strain, in which the target gene is deleted, from the obtained mutants. The method of deleting the gene includes a method using DNA recombination technology. The DNA recombination technology may be accomplished by, for example, injecting a nucleotide sequence or vector including a nucleotide sequence having homology to a target gene into the microorganism to cause homologous recombination. In addition, the injected nucleotide sequence or vector may include a dominant selection marker, but is not limited thereto.

In addition, the method of modifying an expression regulatory sequence may be performed by inducing a modification in the expression regulatory sequence through deletion, insertion, conservative or non-conservative substitution, or a combination thereof so as to further weaken the activity of the expression regulatory sequence or by replacing the sequence with a nucleic acid sequence having a weaker activity. The expression regulatory sequence may include a promoter, an operator sequence, a sequence encoding a ribosome-binding domain, and a sequence for regulating the termination of transcription and translation, but is not limited thereto.

Furthermore, the method of modifying the gene sequence on the chromosome may be performed by inducing a modification in the sequence through deletion, insertion, conservative or non-conservative substitution, or a combination thereof so as to further weaken the activity of the polypeptide; or by replacing the sequence with a gene sequence modified to have a weaker activity or a gene sequence modified to have no activity at all, but is not limited thereto.

However, the above description is only an example, and the method is not limited thereto, and the microorganism may be a microorganism in which expression of genes encoding enzymes of various known biosynthetic pathways that increase fat content is promoted, enzymes of related pathways are inactivated, or an enzyme in a pathway that consumes an intermediate, cofactor, or energy source in the fat biosynthetic pathways is inactivated. The microorganism with the increased fat content may be prepared by applying various known methods.

With respect to the objects of the present disclosure, the microorganism of the present disclosure may be any microorganism, as long as it is a microorganism in which the activity of phosphatidylethanolamine N-methyltransferase (PEMT) or phospholipid methyltransferase is inactivated and the fat content increased, as compared with the non-modified microorganism or the wild-type.

As used herein, the term "non-modified microorganism" may refer to a native strain itself or a microorganism in which the activity of phosphatidylethanolamine *N*-methyltransferase or phospholipid methyltransferase is not reduced or inactivated, but is not limited thereto. The "non-modified microorganism" may be used interchangeably with "a strain before modification", "a microorganism before modification", "a non-variant strain", "a non-modified strain", "a non-variant microorganism", "a non-modified microorganism", or "a reference microorganism".

As used herein, the "wild-type" refers to a universal phenotype originally possessed when a new variant is observed in a species due to mutation in the genotype.

The wild-type strain of the present disclosure may be a strain of the genus *Yarrowia.* The "genus *Yarrowia"* is a fungal genus in the family Dipodascaceae. The genus is a yeast capable of utilizing unusual carbon sources, such as hydrocarbons, and may be monotypic. The strain of the genus *Yarrowia* may be specifically *Yarrowia bubula, Yarrowia deformans*, *Yarrowia lipolytica, Yarrowia porcina, Yarrowia yakushimensis,* and more specifically *Yarrowia lipolytica,* but is not limited thereto.

The "variant strain" of the present disclosure refers to a strain having a new phenotype, when a new variant is observed in a microbial strain due to mutation in the genotype.

The "variant strain of the genus *Yarrowia"* of the present disclosure may be a strain of the genus *Yarrowia,* in which the activity of phosphatidylethanolamine *N*-methyltransferase is inactivated, the activity of phospholipid methyltransferase is inactivated, or the activities of phosphatidylethanolamine *N*-methyltransferase and phospholipid methyltransferase are inactivated.

Further, it may be a strain of the genus *Yarrowia,* in which the *cho2* gene encoding phosphatidylethanolamine *N*-methyltransferase, the *opi3* gene encoding phospholipid methyltransferase, or a combination thereof is deleted, but is not limited thereto.

Specifically, with respect to the objects of the present disclosure, the variant strain of the genus *Yarrowia* is not limited, as long as it is a variant strain in which the activity of phosphatidylethanolamine *N*-methyltransferase or phospholipid methyltransferase is inactivated, and thus the fat content in the strain is increased, as compared with the wild-type.

As used herein, the "microorganism having the increased fat content, as compared with the wild-type" may be used interchangeably with "a fat-producing microorganism", "a microorganism having ability to produce a fat", "a microorganism for fat production", "a triacylglycerol (TAG)-producing microorganism", "a microorganism having ability to produce triacylglycerol (TAG)", or "a microorganism for triacylglycerol (TAG) production".

The variant strain of the genus *Yarrowia* may be a choline auxotrophic strain provided with choline auxotrophy. The "choline" is colorless and strongly alkaline, and is a component of phospholipids, acetyl choline, B vitamins, *etc.* It is synthesized from the route of choline synthesis via serine and ethanolamine in living organisms, and is also chemically synthesized with trimethylamine and ethylene oxide.

With respect to the objects of the present disclosure, when the strain, which is a choline auxotrophic strain, is cultured in a medium containing choline, it is possible to recover the growth of the strain, and at the same time, to increase the fat content in the strain.

As used herein, the term "fat", which is a kind of lipid, is an ester in which three fatty acids are bound with one glycerol, and is a representative organic substance. Fat is contained in living organisms and may be used as an energy source. Specifically, the fat may be triacylglycerol (TAG), but is not limited thereto.

The "triacylglycerol (TAG)", which is a kind of fat, is a triglyceride having a structure in which three fatty acid molecules are bonded to one glycerin molecule via an ester linkage. In order to increase the TAG content, a strategy for enhancing the TAG biosynthetic pathway, inhibiting TAG degradation, or inhibiting beta-oxidation may be used. The term "fat" may be used interchangeably with "TAG" or "triacylglycerol". In addition, TAG may be a precursor of fatty acid derivatives and a form of fat that may be stored for a long time.

Another aspect of the present disclosure provides a cosmetic composition, a food composition, a feed composition, or a medical composition, each including at least one of the variant strain of the genus *Yarrowia* of the present disclosure; a culture of the strain; an extract of the strain; a dry product of the strain; a lysate of the strain; and a fat recovered from at least one of the strain, the culture, the extract, the dry product, and the lysate.

The "genus *Yarrowia"* and the "variant strain of the genus *Yarrowia"* of the present disclosure are the same as described above.

With respect to the objects of the present disclosure, the composition may include the variant strain of the genus *Yarrowia* itself, in which the activity of phosphatidylethanolamine *N*-methyltransferase or phospholipid methyltransferase is inactivated, and thus the fat content in the strain is increased, as compared with the wild-type, and may include the culture, dry product, extract, or lysate of the strain. In addition, the composition may include a fat recovered from any one of the strain of the present disclosure, the culture, the dry product, the extract, or the lysate of the strain. However, the composition may include any form without limitation, as long as it is able to increase the desired TAG content within the scope of the present disclosure.

The "cosmetic composition" of the present disclosure may be formulated into a preparation selected from the group consisting of a solution, an ointment for external use, a cream, a foam, a nutrient lotion, a softening lotion, a pack, a softening water, an emulsion, a makeup base, an essence, a soap, a liquid cleaner, a bath product, a sunscreen cream, a sun oil, a suspension, an emulsion, a paste, a gel, a lotion, a powder, a surfactant-containing cleansing oil, a powder foundation, an emulsion foundation, a wax foundation, a patch, and a spray, but is not limited thereto.

The cosmetic composition may further include one or more cosmetically acceptable carrier which are blended in general skin cosmetics, and as a common component, for example, oil, water, a surfactant, a moisturizer, a lower alcohol, a thickener, a chelating agent, a pigment, a preservative, a fragrance, *etc.* may be appropriately blended, but the carrier is not limited thereto. The cosmetically acceptable carrier to be included in the cosmetic composition of the present disclosure varies depending on the formulation of the cosmetic composition.

The cosmetic composition of the present disclosure may include at least one of the variant strain of the genus *Yarrowia;* the culture of the strain; the extract of the strain; the dry product of the strain; the lysate of the strain; and a fat recovered from at least one of the strain, the culture, the extract, the dry product, and the lysate, and thus may include the increased fat content.

The "food composition" of the present disclosure includes all forms such as a functional food, a nutritional supplement, a health food, a food additive, *etc.,* and the above kinds of the food composition may be prepared into various forms according to a common method known in the art.

The food composition of the present disclosure may include forms of pills, powders, granules, infusions, tablets, capsules, liquids, *etc.,* and examples of the foods to which the composition of the present disclosure is added include various foods, such as beverages, gums, teas, vitamin complexes, health supplement foods, *etc.*

The food composition of the present disclosure may include other additional ingredients such as various herbal medicinal extracts, food supplement additives, or natural carbohydrates, *etc.,* as in common foods. In addition, the food supplement additives may include food additives commonly used in the art, for example, a fragrance agent, a flavoring agent, a coloring agent, a filler, a stabilizer, *etc.*

Examples of the natural carbohydrate include common sugars, such as monosaccharides, e.g., glucose, fructose, *etc.;* disaccharides, e.g., maltose, sucrose, *etc.;* polysaccharides, e.g., dextrin, cyclodextrin, *etc.;* and sugar alcohols such as xylitol, sorbitol, erythritol, *etc.* Also, as a fragrance agent, a natural fragrance agent (e.g., rebaudioside A, glycyrrhizin, *etc.)* and a synthetic fragrance agent (saccharine, aspartame, *etc.)* may be advantageously used.

In addition, the food composition of the present disclosure may include various nutrients, vitamins, minerals (electrolytes), a flavor agent such as a synthetic flavor agent, a natural flavor agent, *etc.,* a coloring agent, an extender (cheese, chocolate, *etc.),* pectic acid and salts thereof, alginic acid and salts thereof, organic acid, a protective colloid thickener, a PH adjuster, a stabilizer, a preservative, glycerin, alcohol, a carbonating agent used for a carbonated drink, *etc.* In addition, the functional food composition of the present disclosure may include flesh that may be used for preparing natural fruit juice, fruit juice drinks, and vegetable drinks. Such components may be used independently or in combination.

The food composition of the present disclosure may be prepared by adding raw materials and ingredients commonly added in the art during the preparation. Additionally, the formulations of the food may be prepared without limitation as long as it may be acknowledged as a food. The food composition of the present disclosure may be prepared into various types of formulations. Unlike general drugs, the food composition of the present disclosure has advantages in that there are no side effects caused by long-term administration, *etc.,* because the food composition employs a food as a raw material, and the food composition of the present disclosure has excellent portability, and thus the food of the present disclosure may be consumed as a supplement.

The food composition of the present disclosure may include at least one of the variant strain of the genus *Yarrowia;* the culture of the strain; the extract of the strain; the dry product of the strain; the lysate of the strain; and a fat recovered from at least one of the strain, the culture, the extract, the dry product, and the lysate, and thus may include an increased fat content.

The "feed composition" of the present disclosure refers to any natural or artificial diet, meal, *etc.,* or components of such meals intended or suitable for being eaten, taken in, or digested by animals. The feed composition may be prepared into various types of feeds known in the art, and specifically, may include concentrated feeds, bulky feeds, and/or specialized feeds.

A kind of the feed is not particularly limited, and any feed generally used in the art may be used. Non-limiting examples of the feed may include plant-based feeds, such as grains, nuts, food by-products, seaweeds, fibers, drug by-products, fats and oils, starches, gourds, grain by-products, *etc.;* and animal-based feeds such as proteins, inorganic matters, fats and oils, minerals, single cell proteins, zooplanktons, foods, *etc.* These may be used alone or in a mixture of two or more thereof.

The feed composition of the present disclosure may include at least one of the variant strain of the genus *Yarrowia;* the culture of the strain; the extract of the strain; the dry product of the strain; the lysate of the strain; and a fat recovered from at least one of the strain, the culture, the extract, the dry product, and the lysate, and thus may include an increased fat content.

The "medical composition" of the present disclosure refers to those prepared for the purpose of preventing or treating a disease, and may be used after being respectively formulated into various forms according to a common method. For example, the medical composition may be formulated into oral dosage forms such as powders, granules, tablets, capsules, suspensions, emulsions, syrups, *etc.,* and may be formulated in the form of preparations for external use, suppositories, and sterile injectable solutions.

In addition, the medical composition may be prepared by further including pharmaceutically acceptable carriers, such as buffers, preservatives, analgesics, solubilizers, isotonic agents, stabilizers, bases, excipients, lubricants, *etc.* known in the art, according to each formulation.

Meanwhile, the medical composition of the present disclosure is administered in a pharmaceutically effective amount. As used herein, the term "pharmaceutically effective amount" means an amount which is sufficient to treat diseases at a reasonable benefit/risk ratio applicable to any medical treatment and does not cause any adverse effect. The effective dosage level may be determined by those skilled in the art, depending on factors, including a patient's health conditions, severity, drug activity, drug sensitivity, administration method, administration time, administration route, excretion rate, duration of treatment, drugs used in combination or used concurrently, and other factors well known in the medical field.

The medical composition of the present disclosure may include at least one of the variant strain of the genus *Yarrowia;* the culture of the strain; the extract of the strain; the dry product of the strain; the lysate of the strain; and a fat recovered from at least one of the strain, the culture, the extract, the dry product, and the lysate, and thus may include an increased fat content.

With respect to the objects of the present disclosure, the increased fat content in the strain may be an increased TAG content, but is not limited thereto.

Still another aspect of the present disclosure provides a method of increasing a fat in a strain, including: culturing the variant strain of the genus *Yarrowia* of the present disclosure.

The "genus *Yarrowia",* the "variant strain of the genus *Yarrowia",* and the "fat" are the same as described above.

As used herein, the "culturing" refers to growing the variant strain of the genus *Yarrowia* under appropriately controlled environmental conditions. The culturing process of the present disclosure may be achieved according to an appropriate medium and culture conditions known in the art. Such a culturing process may be used by easy adjustment of the conditions by those skilled in the art according to the strain being selected.

The culturing the strain may be, but is not particularly limited to, performed in a batch process, a continuous process, a fed-batch process, *etc.* known in the art. In this regard, with respect to the culturing conditions, pH may be adjusted to a suitable pH (e.g., pH 5 to 9, specifically pH 6 to 8, and most specifically pH 6.8) using a basic compound (e.g., sodium hydroxide, potassium hydroxide, or ammonia) or an acidic compound (e.g., phosphoric acid or sulfuric acid), but is not particularly limited thereto. Additionally, aerobic conditions may be maintained by introducing oxygen or an oxygen-containing gas mixture to the culture. The culturing temperature may be maintained at 20°C to 45°C, and specifically 25°C to 40°C for about 10 hours to 160 hours, but is not limited thereto.

As used herein, the "medium" refers to a culture medium which includes a substance obtained by mixing nutrients required for culturing the variant strain of the genus *Yarrowia* as a main component, and/or a product obtained after culturing. The medium and other culture conditions used in culturing the microorganism of the present disclosure are not particularly limited, as long as the medium is a medium commonly used in culturing the microorganism, but the microorganism of the present disclosure may be cultured in a common medium containing appropriate carbon sources, nitrogen sources, phosphorous sources, inorganic compounds, amino acids and/or vitamins, *etc.,* under aerobic conditions while adjusting temperature, pH, *etc.*

As a carbon source for the culture medium used in the present disclosure, sugars and carbohydrates (e.g., glucose, sucrose, lactose, fructose, galactose, mannose, maltose, arabinose, xylose, molasses, starch, and cellulose), oils and fats (*e.g.,* soybean oil, sunflower seed oil, peanut oil, and coconut oil), fatty acids (*e.g.,* palmitic acid, stearic acid, and linoleic acid), alcohols (*e.g.,* glycerol and ethanol), organic acids (*e.g.,* acetic acid), *etc.* may be used alone or in combination. Specifically, the carbon source may be one or more selected from the group consisting of glucose, fructose, maltose, galactose, mannose, sucrose, arabinose, xylose, and glycerol, but is not limited thereto. Further, the culture medium used in the present disclosure may include, as the carbon source, glucose at a concentration of 10 g/L to 50 g/L, 10 g/L to 40 g/L, 20 g/L to 50 g/L, 20 g/L to 40 g/L, or 25 g/L to 35 g/L, but is not limited thereto.

A nitrogen source for the culture medium used in the present disclosure may be classified into an organic nitrogen source or an inorganic nitrogen source, but the organic nitrogen source or the inorganic nitrogen source may be used alone or in a mixture. Specifically, the nitrogen source may be an organic nitrogen source selected from the group consisting of a yeast extract, a beef extract, peptone, and tryptone, or an inorganic nitrogen source selected from the group consisting of ammonium acetate, ammonium nitrate, ammonium chloride, ammonium sulfate, sodium nitrate, urea, and monosodium glutamate (MSG). Further, in the culture medium used in the present disclosure, examples of the nitrogen source may include a yeast extract, ammonium sulfate, sodium nitrate, and MSG, but are not limited thereto.

The yeast extract may be included in the culture medium at a concentration of 0.1 g/L to 10 g/L, 0.5 g/L to 10 g/L, 0.5 g/L to 7 g/L, 0.5 g/L to 5 g/L, 0.5 g/L to 3 g/L, 0.5 g/L to 2 g/L, or 0.5 g/L to 1.5 g/L, the ammonium sulfate may be included in the culture medium at a concentration of 1 g/L to 5 g/L, 1 g/L to 4 g/L, 2 g/L to 5 g/L, or 2 g/L to 4 g/L, the sodium nitrate may be included in the culture medium at a concentration of 0.1 g/L to 10 g/L, 0.5 g/L to 9 g/L, 1 g/L to 9 g/L, 2 g/L to 9 g/L, 3 g/L to 9 g/L, 5 g/L to 9 g/L, or 7 g/L to 9 g/L, and the MSG may be included in the culture medium at a concentration of 0.1 g/L to 2 g/L, 0.1 g/L to 1.5 g/L, 0.5 g/L to 2 g/L, or 0.5 g/L to 1.5 g/L, but these are not limited thereto.

The culturing the variant strain of the genus *Yarrowia* of the present disclosure may be culturing the strain in a medium containing choline. Specifically, a concentration of choline in the medium containing choline of the present disclosure may be 0.05 mM to 5 mM, 0.1 mM to 5 mM, 0.1 mM to 3 mM, 0.1 mM to 2 mM, or 0.1 mM to 1 mM, but is not limited thereto.

With respect to the objects of the present disclosure, when the strain, which is a choline auxotrophic strain, is cultured in a medium containing choline, it is possible to recover the growth of the strain, and at the same time, to increase the fat content in the strain.

With respect to the objects of the present disclosure, the medium may have an optimal C/N ratio in order to increase the fat content in the strain.

As used herein, the "C/N ratio (carbon-to-nitrogen ratio; C:N ratio)" refers to a ratio of the mass of carbon to the mass of nitrogen in a medium. As the ratio increases, the nitrogen source in the medium may decrease, and accordingly, the cell concentration may decrease due to growth inhibition.

As the C/N ratio in the medium of the present disclosure increases, OD value may decrease, and the intracellular fat content may increase. Thus, for the maximum productivity of fat, a preferred C/N ratio in the medium is important.

The C/N ratio showing the maximum fat productivity in the strain of the present disclosure may be calculated by OD * fat content value, and the C/N ratio in the medium may be 10 to 120, specifically 30 to 90, more specifically 40 to 80, and much more specifically 50 to 70, but is not limited thereto.

The preferred C/N ratio in the medium may effectively increase the productivity of fat while preventing the growth inhibition of the strain of the present disclosure.

Still another aspect of the present disclosure provides a method of preparing a fat, including: culturing the variant strain of the genus *Yarrowia* of the present disclosure, in which activity of phosphatidylethanolamine *N-*methyltransferase (PEMT) or phospholipid methyltransferase is inactivated.

The "genus *Yarrowia",* the "variant strain of the genus *Yarrowia",* the "culturing", and the "fat" of the present disclosure are the same as described above.

The culturing the strain may be culturing the strain in a medium containing choline.

The concentration of choline in the medium containing choline of the present disclosure may be 0.05 mM to 5 mM, 0.1 mM to 5 mM, 0.1 mM to 3 mM, 0.1 mM to 2 mM, or 0.1 mM to 1 mM.

The C/N ratio in the medium may be 10 to 120, specifically 30 to 90, more specifically 40 to 80, and much more specifically 50 to 70, but is not limited thereto.

With respect to the objects of the present disclosure, when the strain, which is a choline auxotrophic strain, is cultured in a medium containing choline, it is possible to recover the growth of the strain, and at the same time, to increase the fat content in the strain, and thus it is easy to prepare the fat.

The method of preparing a fat of the present disclosure may further include preparing the microorganism of the present disclosure, preparing a medium for culturing the strain, or a combination thereof (in any order), for example, prior to the culturing.

The method of preparing a fat of the present disclosure may further include recovering the fat from the strain, the culture thereof, the extract thereof, the dry product thereof, or the lysate thereof, after culturing the variant strain of the genus *Yarrowia.* In other words, the fat may be collected from the strain itself or the culture thereof which is produced in the culturing of the present disclosure. For example, centrifugation, filtration, anion-exchange chromatography, crystallization, HPLC, *etc.* may be used, and the target fat may be recovered from the cultured strain or the culture thereof, the dry product thereof, or the lysate thereof using an appropriate method known in the art.

Further, the recovering the fat may further include isolation and/or purification, which may be performed using an appropriate method known in the art. Therefore, the recovered fat may be in a purified form or a microbial fermentation liquid containing the fat.

Still another aspect of the present disclosure provides use of the variant strain of the genus *Yarrowia,* in which the activity of phosphatidylethanolamine N-methyltransferase or phospholipid methyltransferase is inactivated; the culture of the strain; the extract of the strain; the dry product of the strain; the lysate of the strain; the cosmetic composition, the food composition, the feed composition, or the medical composition, each including at least one of the strain, the culture, the extract, the dry product, and the lysate, and the fat recovered from at least one of the strain, the culture, the extract, the dry product, and the lysate, in the fat production.

The "phosphatidylethanolamine *N*-methyltransferase", the "phospholipid methyltransferase", the "genus *Yarrowia",* the "variant strain of the genus *Yarrowia",* the "culturing", the "fat", the "cosmetic composition", the "food composition", the "feed composition", and the "medical composition" of the present disclosure are the same as described above.

### [Mode for Invention]

Hereinafter, the present disclosure will be described in more detail with reference to exemplary embodiments. However, these embodiments are only for more specifically illustrating the present disclosure, and it will be apparent to those of ordinary skill in the art that the scope of the present disclosure is not limited to these embodiments.

### Example 1. Preparation of Wild-Type Yeast-Based Deficient Strain

To prepare an auxotrophic strain for ethanolamine or choline, a strain was prepared in which a CHO2- or OPI3-encoding gene was deleted, based on a wild-type yeast strain, *Yarrowia lipolytica* POlf (ATCC MYA-2613).

### 1-1. Preparation of cho2-deficient strain ("CC08-0162")

To prepare a cassette capable of deleting the *cho2* gene on the chromosome of *Yarrowia lipolytica,* a polynucleotide sequence of SEQ ID NO: 1 and an amino acid sequence of SEQ ID NO: 2 of *cho2* (YALI0E06061g) were obtained based on nucleotide sequences registered at KEGG (Kyoto Encyclopedia of Genes and Genomes).

PCR was performed using a genomic DNA of *Yarrowia lipolytica* PO1f as a template and primers of SEQ ID NOs: 3 and 4, SEQ ID NOs: 7 and 8, and SEQ ID NOs: 9 and 10, respectively. PCR was also performed using a URA3 auxotrophic marker as a template and primers of SEQ ID NOs: 5 and 6. PCR was performed for 35 cycles under conditions consisting of denaturation at 95°C for 1 minute; annealing at 55°C for 1 minute; and polymerization at 72°C for 1 minute and 30 seconds.

As a result, 5' UTR of 1,055 bp, 5' UTR_RP of 638 bp, 3' UTR of 1050 bp, and URA3 of 1,569 bp were obtained. The DNA fragments amplified by PCR were prepared into one ***cho2*-deficient** cassette through overlap extension PCR, and design of the cassette was performed in the order of 5' UTR-URA3-5' UTR_RP-3' UTR.

The ***cho2*-deficient** cassette was transformed into *Yarrowia lipolytica* PO1f by heat shock and selected on a solid medium containing no uracil. The primary strain thus selected was subjected to a second crossover to prepare a *cho2* gene-deficient strain. The deficient strain thus prepared was designated as CC08-0162 and deposited under the Budapest Treaty in the Korean Culture Center of Microorganisms (KCCM), an international depository authority, on February 20, 2020, under Accession No. KCCM12672P.

**[Table 1]**

| SEQ ID NO: | Primer name | Sequence (5-3') |
|---|---|---|
| 3 | CHO2_5'_For | GTACCCGGGGATCCTCTAGAGTCATCCGCAAACACAACAC |
| 4 | CHO2_5'_Rev | GCAATGACGAGTCAGACAGGGGTGTTTGTGGAAGCTGGTG |
| 5 | CHO2_URA_For | CACCAGCTTCCACAAACACCCCTGTCTGACTCGTCATTGC |
| 6 | CHO2_URA_Rev | CGCATTCTGCGTACATTTTGCTGGTGGTATTGTGACTGGG |
| 7 | CHO2_5'RP_For | CCCAGTCACAATACCACCAGCAAAATGTACGCAGAATGCG |
| 8 | CHO2_5'RP_Rev | CAGATATGCTCTCTGCAAACGGTGTTTGTGGAAGCTGGTG |
| 9 | CHO2_3'_For | CACCAGCTTCCACAAACACCGTTTGCAGAGAGCATATCTG |
| 10 | CHO2_3'_Rev | GCAGGTCGACTCTAGAGAGAGGTCTGTTCACAACATCGGC |

### 1-2. Preparation of opi3-deficient strain ("CC08-0123")

To prepare a cassette capable of deleting the *opi3* gene on the chromosome of *Yarrowia lipolytica,* a polynucleotide sequence of SEQ ID NO: 11 and an amino acid sequence of SEQ ID NO: 12 of *opi3* (YALI0E12441g) were obtained based on nucleotide sequences registered at KEGG (Kyoto Encyclopedia of Genes and Genomes).

PCR was performed using the genomic DNA of *Yarrowia lipolytica* PO1f as a template and primers of SEQ ID NOs: 13 and 14, SEQ ID NOs: 17 and 18, and SEQ ID NOs: 19 and 20, respectively. PCR was also performed using the URA3 auxotrophic marker as a template and primers of SEQ ID NOs: 15 and 16. PCR was performed for 35 cycles under conditions consisting of denaturation at 95°C for 1 minute; annealing at 55°C for 1 minute; and polymerization at 72°C for 1 minute and 30 seconds.

As a result, 5' UTR of 980 bp, 5' UTR_RP of 730 bp, 3' UTR of 1,001 bp, and URA3 of 1,569 bp were obtained. The DNA fragments amplified by PCR were prepared into one ***opi3*-deficient** cassette through overlap extension PCR, and design of the cassette was performed in the order of 5' UTR-URA3-5' UTR_RP-3' UTR.

The ***opi3*-deficient** cassette was transformed into *Yarrowia lipolytica* PO1f by heat shock and selected on a solid medium containing no uracil. The primary strain thus selected was subjected to a second crossover to prepare an *opi3* gene-deficient strain. The deficient strain thus prepared was designated as CC08-0123 and deposited under the Budapest Treaty at the Korean Culture Center of Microorganisms (KCCM), an international depository authority, on February 20, 2020, under Accession No. KCCM12673P.

**[Table 2]**

| SEQ ID NO: | Primer name | Sequence (5-3') |
|---|---|---|
| 13 | OPI3_5'_For | GTACCCGGGGATCCTCTAGACACCAACATTCGACATGGAC |
| 14 | OPI3_5'_Rev | GCAATGACGAGTCAGACAGGGAGTTTTCCAGAGAGCCAAC |
| 15 | OPI3_URA_For | GTTGGCTCTCTGGAAAACTCCCTGTCTGACTCGTCATTGC |
| 16 | OPI3_URA_Rev | CAGTCCTTAATCAACGGTGGCTGGTGGTATTGTGACTGGG |
| 17 | OPI3_5'RP_For | CCCAGTCACAATACCACCAGCCACCGTTGATTAAGGACTG |
| 18 | OPI3_5'RP_Rev | CGTAGGCCGTTTTCTGTTGCGAGTTTTCCAGAGAGCCAAC |
| 19 | OPI3_3'_For | GTTGGCTCTCTGGAAAACTCGCAACAGAAAACGGCCTACG |
| 20 | OPI3_3'_Rev | GCAGGTCGACTCTAGAGAGTGCCGTCTCGATTGTCACAGG |

### 1-3. Preparation of cho2- and opi3-codeficient strain ("CC08-0183")

To examine the effect of *cho2* and *opi3* codeficiency in *Yarrowia lipolytica* PO1f, a *cho2-* and *opi3*-codeficient strain was prepared in the same manner as in Examples 1-1 and 1-2, and the strain, in which the respective genes were deleted in combination, were designated as CC08-0183 and deposited under the Budapest Treaty at the Korean Culture Center of Microorganisms (KCCM), an international depository authority, on February 20, 2020, under Accession No. KCCM12671P.

### Example 2. Evaluation of Growth and Fat Accumulation of Wild-Type Strain-Based Deficient Strains

The growth and fat accumulation of the deficient strains CC08-0162, CC08-0123, and CC08-0183 prepared in Example 1 were evaluated.

### 2-1. Evaluation according to medium

The two kinds of strains and the control group (PO1f) were seeded in a 250 mL corner-baffle flask containing 50 mL of YPD or YLMM1 (*Yarrowia lipolytica* minimal media 1) at an initial OD of 0.01, and cultured with shaking at 250 rpm and 30°C for 72 hours. In YLMM1, 0.3 mM choline chloride (CL) was added, if necessary. Compositions of the YPD and fat medium 1 (YLMM1) are as follows.

### <YPD>

20 g/L glucose, 10 g/L yeast extract (manufactured by BD, Bacto yeast extract, 0.38% choline in Bacto yeast extract), 0.5 g/L uracil, 20 g/L Bacto peptone

### <YLMM1 (pH 7.2)>

40 g/L glucose, 1.7 g/L yeast nitrogen base without amino acids and ammonium sulfate, 0.5 g/L uracil, and 2.5 g/L ammonium sulfate were dissolved in 0.1 M sodium phosphate buffer (pH 7.2).

After the culturing was terminated, the culture medium was centrifuged to discard the supernatant, and cells remaining in the lower layer were dried in a dry oven at 60°C for 24 hours, followed by crude fat extraction. Crude fat extraction was performed according to the feed standard analysis method (feed standard analysis method. 2001. National Livestock Research Institute, Rural Development Administration), and the analyzed OD and the intracellular crude fat content are shown in Table 3 below. The results in Table 3 are the results of experiments performed in triplicate, and the increase in the crude fat content was evaluated by mean values thereof.

**[Table 3]**

| Medium | Strain | OD₆₀₀ | | | | Crude fat content [%] | | | |
|---|---|---|---|---|---|---|---|---|---|
| | | Batch 1 | Batch 2 | Batch 3 | Mean | Batch 1 | Batch 2 | Batch 3 | Mean |
| YPD | PO1f | 60.8 | 63.2 | 62.9 | 62.3 | 9.7 | 9.4 | 9.4 | 9.5 |
| | CC08-O162 | 61.5 | 60.9 | 62.7 | 61.7 | 11.2 | 10.7 | 11.4 | 11.1 |
| | CC08-O123 | 62.5 | 62.4 | 61.4 | 62.1 | 11.0 | 10.7 | 10.7 | 10.8 |
| | CC08-O183 | 61.7 | 61.5 | 61.9 | 61.7 | 11.1 | 11.0 | 11.2 | 11.1 |
| YIMM1 | PO1f | 47.2 | 46.1 | 47.7 | 47.0 | 10.9 | 11.5 | 12.4 | 11.6 |
| | PO1f (w/ CL) | 46.6 | 47.9 | 47.2 | 47.2 | 11.9 | 11.1 | 11.5 | 11.5 |
| | CC08-O162 | 10.8 | 11.9 | 13.6 | 12.1 | 15.0 | 14.6 | 15.1 | 14.9 |
| | CC08-O162 (w/ CL) | 44.4 | 45.0 | 48.3 | 45.9 | 16.4 | 16.1 | 16.4 | 16.3 |
| | CC08-O123 | 18.0 | 16.6 | 17.3 | 17.3 | 14.2 | 14.4 | 14.9 | 14.5 |
| | CC08-O123 (w/ CL) | 45.7 | 46.5 | 46.4 | 46.2 | 16.1 | 15.6 | 16.3 | 16.0 |
| | CC08-O183 | 12.1 | 12.1 | 12.4 | 12.2 | 14.9 | 15.0 | 15.1 | 15.0 |
| | CC08-O183 (w/ CL) | 45.8 | 46.1 | 45.9 | 45.9 | 16.3 | 16.5 | 16.2 | 16.3 |

As shown in Table 3, it was confirmed that the crude fat content was increased regardless of the medium in the strains, in which the *cho2* or *opi3* gene was individually or simultaneously deleted from the parent strain PO1f. It was also observed that the crude fat content increased when the same strain was cultured in YLMM1 than when cultured in YPD. The increase was about 17% in the ***cho2*-deficient** strain and about 14% in the ***opi3*-deficient** strain, as compared with PO1f, in the YPD medium. In contrast, the increase was about 28% in the ***cho2*-deficient** strain and about 25% in the ***opi3*-deficient** strain, as compared with PO1f, in the YLMM1 medium, indicating that the increase of the fat content was larger in YLMM1 than in YPD. In addition, in the strain in which the *cho2* and *opi3* genes were simultaneously deleted, an increase of the fat content similar to that of the ***cho2*-deficient** strain was observed.

However, unlike YPD, YLMM1 contained no choline, and thus the pathway to synthesize phosphatidylcholine from phosphatidyl ethanolamine was blocked, and the ***cho2*-deficient** strain, the ***opi3*-deficient** strain, and the *cho2-* and *opi3-*codeficient strain provided with choline auxotroph showed growth inhibition in YLMM1.

Thus, 0.3 mM choline was added to the medium for growth recovery, and it was confirmed that when choline was added to the medium, the growth of the choline auxotrophic strain was recovered as much as in the control group, and the crude fat content in the strain was also increased by about 10%, as compared with no addition of choline.

The growth and fat accumulation of the *cho2-* and ***opi3*-deficient** strains prepared based on *Yarrowia lipolytica* PO1f yeast showed a similar tendency to those of the budding yeast *Saccharomyces cerevisiae,* but the strains are different from *Saccharomyces cerevisiae* in that when choline was added, their growth recovered and the fat content was also increased, which is a characteristic of the oleaginous yeast, *Yarrowia lipolytica.*

### 2-2. Evaluation of growth and fat accumulation according to C/N ratio

When culturing was performed in YLMM1 with a C/N ratio of 30, as compared with YPD with a C/N ratio of less than 1, in Example 2-1, the fat content in the strain was confirmed to increase. Accordingly, the three kinds of strains prepared in Example 1 and the control PO1f were cultured in CN test media having different C/N ratios, and the growth and fat accumulation were evaluated according to the C/N ratio. Each strain was seeded in a 250 mL corner-baffle flask containing 50 mL of CN test medium, to which different concentrations of ammonium sulfate were added, at an initial OD of 0.01, and cultured with shaking at 250 rpm and 30°C for 72 hours. The composition of the CN test medium according to the C/N ratio is as follows.

### <CN test medium (pH 7.2)>

40 g/L glucose; 1.7 g/L yeast nitrogen base without amino acids and ammonium sulfate; 0.5 g/L uracil; 0.3 mM choline chloride; 2.5 g/L (C/N ratio of 30), 1.25 g/L (C/N ratio of 60), 0.83 g/L (C/N ratio of 90), or 0.63 g/L (C/N ratio of 120) ammonium sulfate were dissolved in 0.1 M sodium phosphate buffer (pH 7.2).

After the culturing was terminated, the culture medium was centrifuged to discard the supernatant, and cells remaining in the lower layer were dried in a dry oven at 60°C for 24 hours, followed by fat extraction. Crude fat extraction was performed according to the feed standard analysis method (feed standard analysis method. 2001. National Livestock Research Institute, Rural Development Administration), and the analyzed OD and the intracellular crude fat content are shown in Table 4 below. The results in Table 4 are the results of experiments performed in triplicate, and the increase in the fat content was evaluated by mean values thereof.

**[Table 4]**

| C/N ratio | Strain | OD₆₀₀ | | | | Crude fat content [%] | | | |
|---|---|---|---|---|---|---|---|---|---|
| | | Batch1 | Batch 2 | Batch 3 | Mean | Batch 1 | Batch 2 | Batch 3 | Mean |
| 30 | PO1f | 47.2 | 46.1 | 47.7 | 47.0 | 10.9 | 11.5 | 12.4 | 11.6 |
| | CC08-O162 | 44.4 | 45.0 | 48.3 | 45.9 | 16.4 | 16.1 | 16.4 | 16.3 |
| | CC08-O123 | 45.7 | 46.5 | 46.4 | 46.2 | 16.1 | 15.6 | 16.3 | 16.0 |
| | CC08-O183 | 46.2 | 45.9 | 46.0 | 46.0 | 16.1 | 16.3 | 16.2 | 16.2 |
| 60 | PO1f | 44.9 | 45.5 | 45.5 | 45.3 | 12.8 | 12.9 | 13.3 | 13.0 |
| | CC08-O162 | 44.5 | 44.0 | 43.8 | 44.1 | 18.9 | 18.4 | 19.1 | 18.8 |
| | CC08-O123 | 44.9 | 44.6 | 45.2 | 44.9 | 18.0 | 18.8 | 19.3 | 18.7 |
| | CC08-O183 | 43.9 | 44.2 | 44.1 | 44.1 | 19.2 | 18.5 | 18.9 | 18.9 |
| 90 | PO1f | 33.3 | 34.1 | 33.1 | 33.5 | 15.5 | 15.1 | 15.0 | 15.2 |
| | CC08-O162 | 32.0 | 31.7 | 31.1 | 31.6 | 22.2 | 21.8 | 22.3 | 22.1 |
| | CC08-O123 | 32.6 | 32.2 | 32.1 | 32.3 | 21.7 | 21.9 | 22.4 | 22.0 |
| | CC08-O183 | 31.8 | 32.1 | 31.5 | 31.8 | 21.9 | 22.5 | 22.2 | 22.2 |
| 120 | PO1f | 23.7 | 23.9 | 24.4 | 24.0 | 16.7 | 16.4 | 16.7 | 16.6 |
| | CC08-O162 | 23.3 | 23.2 | 23.7 | 23.4 | 24.1 | 24.6 | 24.2 | 24.3 |
| | CC08-O123 | 23.8 | 23.1 | 23.0 | 23.3 | 24.0 | 24.1 | 23.6 | 23.9 |
| | CC08-O183 | 23.4 | 23.6 | 23.1 | 23.4 | 24.3 | 24.0 | 24.1 | 24.1 |

As shown in Table 4, it was confirmed that the fat content was increased regardless of the C/N ratio of the medium in the strains, in which the *cho2* or *opi3* gene was individually or simultaneously deleted from the parent strain PO1f, as in Example 2-1. It was also observed that the increase was at a similar level to be about 43%, regardless of the C/N ratio.

However, as the C/N ratio in the medium increased, the nitrogen source decreased and the OD tended to decrease, whereas the intracellular fat content tended to increase. In order to obtain the C/N ratio showing the maximum productivity, the OD * fat content value was calculated. In all strains, when the C/N ratio was 60, the maximum OD * fat content value was observed. Therefore, it was evaluated that the C/N ratio of 60 is the optimal C/N ratio.

### Example 3. Preparation and Evaluation of Fat-Accumulating SCO023-Based Deficient Strain

### 3-1. Preparation of fat-accumulating strain SCO023

To examine the effect of CHO2- or OPI3-encoding gene deletion on a fat-accumulating strain, a high fat-producing yeast strain was prepared.

To prepare the high fat-producing yeast strain, the TAG degradation pathway was first blocked. The TAG degradation pathway is as follows: TAG is converted into fatty acid (FA) by TGL3 and 4, and the FA is introduced into the peroxisome with the aid of acyl-CoA binding proteins. The introduced FA is acylated by two peroxisomal acyl-CoA Synthases (pxa1, 2), and the acylated FA is desaturated by acyl-CoA oxidases (pox1-6) at the vinyl position. In the desaturated FA-CoA ester, the double bond portion is hydrated by multi-function enzyme 2 (MFE2-C domain, mfe1), and a 3-hydroxyacyl-CoA intermediate is formed. Here, the A/B domain of the MFE2 enzyme acts to oxidize the 3-hydroxyacyl-CoA intermediate to a 3-ketoacyl-CoA intermediate, and the 3-ketoacyl-CoA intermediate is cleaved at the alpha carbon by peroxisomal 3-oxyacyl-thiolase (pot1) to produce acetyl-CoA and fatty acyl-CoA. FA degradation occurs, as the cycle from the pox reaction to the pot1 reaction is repeated (AIMS Bioengineering 2016. 3(4):493-514).

Among the genes, *pox2* (YALI0F10857), *mfe1* (YALI0E15378), and *pot1* (YALI0E18568) were deleted in *Yarrowia lipolytica* PO1f to prevent each enzyme from being expressed. In addition, in order to reduce the number of peroxisomes, the place where beta-oxidation occurs, *pex10* (YALI0C01023), which is a gene involved in the formation of peroxisomes, was deleted to prevent the enzyme from being expressed *(*AIMS Bioengineering 2016. 3(4):493-514). Finally, *mhy1* (YALI0B21582), which is a morphology-related gene, was deleted. If *mhy1* gene was deleted, the carbon flux into amino acid biosynthesis decreased and the carbon flux into fat biosynthesis increased, thereby increasing the intracellular crude fat content (Biochim. Biophys. Acta. 2018. 1863(1):81-90).

To prepare a cassette capable of deleting each gene, a polynucleotide sequence of SEQ ID NO: 21 and an amino acid sequence of SEQ ID NO: 22 of *pox2,* a polynucleotide sequence of SEQ ID NO: 23 and an amino acid sequence of SEQ ID NO: 24 of *mfe1,* a polynucleotide sequence of SEQ ID NO: 25 and an amino acid sequence of SEQ ID NO: 26 of *pex10*, a polynucleotide sequence of SEQ ID NO: 27 and an amino acid sequence of SEQ ID NO: 28 of *pot1,* and a polynucleotide sequence of SEQ ID NO: 29 and an amino acid sequence of SEQ ID NO: 30 of *mhy1* were obtained based on nucleotide sequences registered at KEGG.

Design of each gene-deficient cassette was performed in the order of 5'UTR-URA3-5'UTR_RP-3'UTR, and 5'UTR, 5'UTR_RP, and 3'UTR were subjected to PCR using the genomic DNA of *Yarrowia lipolytica* PO1f as a template and the following primers, respectively; for the preparation of a *pox2-*deficient cassette, primers of SEQ ID NOs: 31 and 32, SEQ ID NOs: 35 and 36, SEQ ID NOs: 37 and 38, for the preparation of an *mfe1*-deficient cassette, primers of SEQ ID NOs: 39 and 40, SEQ ID NOs: 43 and 44, SEQ ID NOs: 45 and 46, for the preparation of a *pex10*-deficient cassette, primers of SEQ ID NOs: 47 and 48, SEQ ID NOs: 51 and 52, SEQ ID NOs: 53 and 54, for the preparation of a *pot1-*deficient cassette, primers of SEQ ID NOs: 55 and 56, SEQ ID NOs: 59 and 60, SEQ ID NOs: 61 and 62, and for the preparation of an *mhy1*-deficient cassette, primers of SEQ ID NOs: 63 and 64, SEQ ID NOs: 67 and 68, SEQ ID NOs: 69 and 70 were used. For URA3, PCR was performed using a URA3 auxotrophic marker as a template and primers of SEQ ID NOs: 33 and 34 for the preparation of the *pox2*-deficient cassette, primers of SEQ ID NOs: 41 and 42 for the preparation of the *mfe1*-deficient cassette, primers of SEQ ID NOs: 49 and 50 for the preparation of the *pex10*-deficient cassette, primers of SEQ ID NOs: 57 and 58 for the preparation of the *pot1*-deficient cassette, and primers of SEQ ID NOs: 65 and 66 for the preparation of the mhy1-deficient cassette.

PCR was performed for 35 cycles under conditions consisting of denaturation at 95°C for 1 minute; annealing at 55°C for 1 minute; and polymerization at 72°C for 1 minute and 30 seconds. As a result, pox2_5' UTR of 1,035 bp, pox2_5' UTR_RP of 720 bp, pox2_3' UTR of 1,010 bp, pox2_URA3 of 1,569 bp, mfe1_5' UTR of 1,003 bp, mfe1_5' UTR_RP of 709 bp, mfe1_3' UTR of 1,036 bp, mfe1_URA3 of 1,569 bp, pex10_5' UTR of 1,032 bp, pex10_5' UTR_RP of 735 bp, pex10_3' UTR of 1,033 bp, pex10_URA3 of 1,569 bp, pot1_5' UTR of 1,036 bp, pot1_5' UTR_RP of 735 bp, pot1_3' UTR of 1,010 bp, pot1_URA3 of 1,569 bp, mhy1_5' UTR of 921 bp, mhy1_5' UTR_RP of 526 bp, mhy1_3' UTR of 1,029 bp, and mhy1_URA3 of 1,569 bp were obtained. The DNA fragments amplified by PCR were prepared into a *pox2-, mfe1-, pex10-, pot1-,* or *mhy1*-deficient cassette through overlap extension PCR.

First, the *pox2*-deficient cassette was transformed into *Yarrowia lipolytica* PO1f by heat shock and selected on a solid medium containing no uracil. The primary strain thus selected was subjected to a second crossover to prepare a *pox2* gene-deficient strain. In the same manner, *mfe1-, pex10-, pot1-,* and *mhy1*-deficient strains were prepared using *mfe1-, pex10-, pot1-,* or *mhy1-*deficient cassettes. The deficient strains thus prepared were designated as "SCO023".

**[Table 5] C**

| SEQ ID NO: | Primer name | Sequence (5-3') |
|---|---|---|
| 31 | POX2_5'_For | GTACCCGGGGATCCTCTAGAGATTCCGCCAAGTGAGACTG |
| 32 | POX2_5'_Rev | GCAATGACGAGTCAGACAGGCGTTGCTTGTGTGATTTTTG |
| 33 | POX2_URA_For | CAAAAATCACACAAGCAACGCCTGTCTGACTCGTCATTGC |
| 34 | POX2_URA_Rev | CGCTTGTCCAGTATGAATAGCTGGTGGTATTGTGACTGGG |
| 35 | POX2_5'RP_For | CCCAGTCACAATACCACCAGCTATTCATACTGGACAAGCG |
| 36 | POX2_5'RP_Rev | CAATAAATACCCGCTTGTCCGTTGCTTGTGTGATTTTTG |
| 37 | POX2_3'_For | CAAAAATCACACAAGCAACGGACAAGCGGGTATTTATTG |
| 38 | POX2_3'_Rev | GCAGGTCGACTCTAGACCAAACAAAGCTGATGACAC |
| 39 | MFE1_5'_For | GTACCCGGGGATCCTCTAGACAAGCCAAAGAGATGTTGAC |
| 40 | MFE1_5'_Rev | GCAATGACGAGTCAGACAGGGTTAATAACGTATCTTCTAC |
| 41 | MFE1_URA_For | GTAGAAGATACGTTATTAACCCTGTCTGACTCGTCATTGC |
| 42 | MFE1_URA_Rev | CAAATCAGCCGTTGGCCTGCCTGGTGGTATTGTGACTGGG |
| 43 | MFE1_5'RP_For | CCCAGTCACAATACCACCAGGCAGGCCAACGGCTGATTTG |
| 44 | MFE1_5'RP_Rev | CACTTGGTCAGATAATAGCGTTAATAACGTATCTTCTAC |
| 45 | MFE1_3'_For | GTAGAAGATACGTTATTAACGCTATTATCTGACCAAGTG |
| 46 | MFE1_3'_Rev | GCAGGTCGACTCTAGATGCTTGAAGGCATATGTGACTG |
| 47 | PEX10_5'_For | GTACCCGGGGATCCTCTAGAGTTTTCGTCTTAGCGTCATG |
| 48 | PEX10_5'_Rev | GCAATGACGAGTCAGACAGGGCCGAGGCAGATTTGGGTTG |
| 49 | PEX10_URA_For | CAACCCAAATCTGCCTCGGCCCTGTCTGACTCGTCATTGC |
| 50 | PEX10_URA_Rev | GAGTCCAGTAATTCTTTCCGCTGGTGGTATTGTGACTGGG |
| 51 | PEX10_5'RP_For | CCCAGTCACAATACCACCAGCGGAAAGAATTACTGGACTC |
| 52 | PEX10_5'RP_Rev | CCTTCCATCCAGACCTCGTCGCCGAGGCAGATTTGGGTTG |
| 53 | PEX10_3'_For | CAACCCAAATCTGCCTCGGCGACGAGGTCTGGATGGAAGG |
| 54 | PEX10_3'_Rev | GCAGGTCGACTCTAGAGCTGACCTATACCAGATCAGACGC |
| 55 | POT1_5'_For | GTACCCGGGGATCCTCTAGACACAATACCCCACAGTGTGC |
| 56 | POT1_5'_Rev | GCAATGACGAGTCAGACAGGGTGTGTCTTGGTTGGATGAG |
| 57 | POT1_URA_For | CTCATCCAACCAAGACACACCCTGTCTGACTCGTCATTGC |
| 58 | POT1_URA_Rev | CAGGCGCTCCCCCATTGGCGCTGGTGGTATTGTGACTGGG |
| 59 | POT1_5'RP_For | CCCAGTCACAATACCACCAGCGCCAATGGGGGAGCGCCTG |
| 60 | POT1_5'RP_Rev | GTTCGATCGCGATTCATTTCGTGTGTCTTGGTTGGATGAG |
| 61 | POT1_3'_For | CTCATCCAACCAAGACACACGAAATGAATCGCGATCGAAC |
| 62 | POT1_3'_Rev | GCAGGTCGACTCTAGAGAGGAGTGCTAAAATTAGCCCTGC |
| 63 | MHY1_5'_For | GTACCCGGGGATCCTCTAGATGTCAGCGAAAGCTCAAAG |
| 64 | MHY1_5'_Rev | GCAATGACGAGTCAGACAGGCAATTCGAGGTCCATTTTGG |
| 65 | MHY1_URA_For | CCAAAATGGACCTCGAATTGCCTGTCTGACTCGTCATTGC |
| 66 | MHY1_URA_Rev | GTGGTGCTTTTGTACTTGTCCTGGTGGTATTGTGACTGGG |
| 67 | MHY1_5'RP_For | CCCAGTCACAATACCACCAGGACAAGTACAAAAGCACCAC |
| 68 | MHY1_5'RP_Rev | GAAGGCGCTCTACCTCTAGTCCAATTCGAGGTCCATTTTG |
| 69 | MHY1_3'_For | CAAAATGGACCTCGAATTGGACTAGAGGTAGAGCGCCTTC |
| 70 | MHY1_3'_Rev | GCAGGTCGACTCTAGACTGTGTTTGATTAGCTCTTCTCAG |

### 3-2. Preparation of SCO023-based cho2-deficient strain ("SCO079")

A *cho2* gene-deficient strain was prepared based on the high fat-producing yeast strain SCO023 prepared in Example 3-1. The *cho2*-deficient cassette prepared in Example 1-1 was transformed into SCO023 by heat shock and selected on a solid medium containing no uracil. The primary strain thus selected was subjected to a second crossover to prepare a *cho2* gene-deficient strain. The deficient strain thus prepared was designated as "SCO079".

### 3-3. Preparation of SCO023-based opi3-deficient strain ("SCO163")

An *opi3* gene-deficient strain was prepared based on the high fat-producing yeast strain SCO023 prepared in Example 3-1. The *opi3*-deficient cassette prepared in Example 1-2 was transformed into SCO023 by heat shock and selected on a solid medium containing no uracil. The primary strain thus selected was subjected to a second crossover to prepare an *opi3* gene-deficient strain. The deficient strain thus prepared was designated as "SCO163".

### 3-4. Flask test

A flask test of the SCO079 and SCO163 strains prepared in Examples 3-2 and 3-3 was performed.

The two kinds of strains and the control group were seeded in a 250 mL corner-baffle flask containing 50 mL of YLMM2, to which different concentrations of choline were added, at an initial OD of 0.01, and cultured with shaking at 250 rpm and 30°C for 72 hours. The composition of the YLMM2 is as follows.

### <YLMM2 (pH 7.2)>

40 g/L glucose, 1.7 g/L yeast nitrogen base without amino acids and ammonium sulfate, 0.5 g/L uracil, and 1.25 g/L ammonium sulfate were dissolved in 0.1 M sodium phosphate buffer (pH 7.2).

After the culturing was terminated, the culture medium was centrifuged to discard the supernatant, and cells remaining in the lower layer were dried in a dry oven at 60°C for 24 hours, followed by fat extraction. Crude fat extraction was performed according to the feed standard analysis method (feed standard analysis method. 2001. National Livestock Research Institute, Rural Development Administration), and the analyzed OD and the intracellular fat content are shown in Table 6 below. The results in Table 6 are the results of experiments performed in triplicate, and the increase in the fat content was evaluated by mean values thereof.

**[Table 6]**

| Strain | Choline concentration [mM] | OD₆₀₀ | | | | Crude fat content [%] | | | |
|---|---|---|---|---|---|---|---|---|---|
| | | Batch 1 | Batch 2 | Batch 3 | Mean | Batch 1 | Batch 2 | Batch 3 | Mean |
| SCO023 | 0.00 | 45.5 | 45.9 | 46.0 | 45.8 | 22.8 | 23.0 | 22.6 | 22.8 |
| | 0.05 | 45.8 | 46.4 | 45.8 | 46.0 | 22.7 | 22.3 | 22.8 | 22.6 |
| | 0.10 | 46.1 | 45.5 | 46.1 | 45.9 | 22.8 | 22.7 | 23.2 | 22.9 |
| | 0.30 | 46.3 | 46.0 | 46.3 | 46.2 | 22.6 | 22.4 | 22.8 | 22.6 |
| | 0.50 | 46.8 | 47.2 | 46.7 | 46.9 | 22.9 | 22.5 | 23.0 | 22.8 |
| | 1.00 | 47.1 | 47.0 | 46.0 | 46.7 | 22.4 | 22.3 | 22.8 | 22.5 |
| SCO079 | 0.00 | 9.8 | 9.6 | 11.2 | 10.2 | 25.3 | 25.2 | 25.7 | 25.4 |
| | 0.05 | 31.7 | 31.4 | 31.7 | 31.6 | 26.5 | 26.1 | 26.0 | 26.2 |
| | 0.10 | 44.0 | 43.9 | 43.5 | 43.8 | 27.9 | 26.7 | 26.7 | 27.1 |
| | 0.30 | 46.6 | 44.4 | 44.9 | 45.3 | 27.5 | 27.7 | 27.6 | 27.6 |
| | 0.50 | 45.2 | 47.1 | 44.8 | 45.7 | 26.8 | 26.3 | 26.1 | 26.4 |
| | 1.00 | 45.4 | 46.3 | 46.6 | 46.1 | 25.7 | 26.0 | 26.3 | 26.0 |
| SCO163 | 0.00 | 11.4 | 10.5 | 12.0 | 11.3 | 25.9 | 25.1 | 24.9 | 25.3 |
| | 0.05 | 33.5 | 31.6 | 31.8 | 32.3 | 26.0 | 26.0 | 25.7 | 25.9 |
| | 0.10 | 43.7 | 44.4 | 44.5 | 44.2 | 26.1 | 26.4 | 26.1 | 26.2 |
| | 0.30 | 44.6 | 45.8 | 44.3 | 44.9 | 26.9 | 26.0 | 27.2 | 26.7 |
| | 0.50 | 45.5 | 44.7 | 45.1 | 45.1 | 26.3 | 25.6 | 26.4 | 26.1 |
| | 1.00 | 46.0 | 45.8 | 44.7 | 45.5 | 26.0 | 26.1 | 25.6 | 25.9 |

As shown in Table 6, the strain, in which the *cho2* or *opi3* gene was deleted from the parent strain SCO023, showed the fat content equal or similar to the parent strain SCO023 in the medium containing choline at a concentration of 0 mM or 0.055 mM, but OD was much reduced. When choline was added at a concentration of 0.1 mM or more to the medium, the OD was recovered to the level of the control group, and the effect of increasing the fat content in the strain was also observed. When 0.1 mM to 1 mM choline was added to the medium, SCO079 showed a 16% to 22% increase in the crude fat content, and SCO163 showed a 14% to 18% increase in the crude fat content, as compared with SCO023. However, in the SCO023 strain, where the PEMT pathway remained, there was no change in the crude fat content, regardless of the choline concentration in the medium.

As a result, it was confirmed that when an appropriate amount of choline was added to the strain, in which the *cho2* or *opi3* gene on the PEMT pathway was deleted, the growth of the strain recovered and the fat content in the strain was increased. However, it was confirmed that the increase in the fat content was relatively low when choline was added after the *cho2* and *opi3* genes were deleted based on the fat accumulating strain SCO023, as compared with the case where the *cho2* and *opi3* genes were deleted based on the wild-type strain.

### Example 4. Preparation and Evaluation of Fat-Accumulating SCO028-Based Deficient Strain

### 4-1. Preparation of fat-accumulating strain SCO028

To examine the effect of CHO2- or OPI3-encoding gene deletion on a strain having a higher fat content than the strain prepared in Example 3-1, another high fat-producing yeast strain was prepared.

It is known that when acyl-CoA:diacylglycerol acyltransferase isozyme I (DGA1), which is a terminal enzyme of the fat biosynthetic pathway, is overexpressed, based on *mfe1-* and *pex10*-deficient strains, the fat content is increased (Nat. Commun. 2014. 5:3131). Therefore, a promoter of *dga1* (YALI0E32769) was replaced with a TEFINt promoter, based on the **SCO023** strain prepared in Example 3-1, in which the fat degradation pathway was blocked and the carbon flux into fat biosynthesis increased (METAB ENG 2015. 29:56-65). A process of preparing a cassette for the promoter replacement is as follows.

To prepare the cassette capable of replacing the promoter of *dga1* gene, a polynucleotide sequence of SEQ ID NO: 71 and an amino acid sequence of SEQ ID NO: 72 of *dga1* were obtained based on the nucleotide sequence registered at KEGG. Design of the cassette for replacing the *dga1* promoter was performed in the order of 5' UTR-TEFINt1-URA3-TEFINt2-gene(dga1), and for 5' UTR, TEFINt1, and TEFINt2, PCR was performed using the genomic DNA of *Yarrowia lipolytica* PO1f as a template and primers of SEQ ID NOs: 73 and 74, SEQ ID NOs: 75 and 76, SEQ ID NOs: 79 and 80, and SEQ ID NOs: 81 and 82, respectively. Further, PCR was performed using the URA3 auxotrophic marker as a template and primers of SEQ ID NOs: 77 and 78. PCR was performed for 35 cycles under conditions consisting of denaturation at 95°C for 1 minute; annealing at 55°C for 1 minute; and polymerization at 72°C for 1 minute and 30 seconds. As a result, dga1_5' UTR of 1,539 bp, dga1_TEFINt1 of 573 bp, dga1_TEFINt2 of 571 bp, dga1_gene of 1,539 bp, and dga1_URA3 of 1,574 bp were obtained. The DNA fragments amplified by PCR were subjected to overlap extension PCR to prepare the cassette for replacing the *dga1* promoter.

The cassette for replacing the *dga1* promoter was transformed into SCO023 by heat shock and selected on a solid medium containing no uracil. The primary strain thus selected was subjected to a second crossover to prepare a strain in which the *dga1* promoter was replaced with the TEFINt promoter. The deficient strain thus prepared was designated as "SCO028".

**[Table 7]**

| SEQ ID NO: | Primer name | Sequence (5'-3') |
|---|---|---|
| 73 | DGA1_5'_For | GTACCCGGGGATCCTCTAGAGACGAGAAACAAATCATGTG |
| 74 | DGA1_5'_Rev | CGCCGCCAACCCGGTCTCTAGCTTTTGTTTTGTGTGACTTG |
| 75 | DGA1_TEF1_For | CAAGTCACACAAAACAAAAGCTAGAGACCGGGTTGGCGGCG |
| 76 | DGA1_TEF1_Rev | GACGAGTCAGACAGGAGGCACTGCGGTTAGTACTGCAAAAAG |
| 77 | DGA1_URA_For | CTTTTTGCAGTACTAACCGCAGTGCCTCCTGTCTGACTCGTC |
| 78 | DGA1_URA_Rev | CGCCGCCAACCCGGTCTCTTGGTGGTATTGTGACTGGG |
| 79 | DGA1_TEF2_For | CCCAGTCACAATACCACCAAGAGACCGGGTTGGCGGCG |
| 80 | DGA1_TEF2_Rev | GTAGTATTGTGAGTCGATAGTCTGCGGTTAGTACTGCAAAAAG |
| 81 | DGA1_dga1_For | CTTTTTGCAGTACTAACCGCAGACTATCGACTCACAATACTAC |
| 82 | DGA1_dga1_Rev | GCCTGCAGGTCGACTCTAGATTACTCAATCATTCGGAACTC |

### 4-2. Preparation of SCO028-based cho2-deficient strain ("SCO048")

A *cho2* gene-deficient strain was prepared based on the high fat-producing yeast strain SCO028 prepared in Example 4-1. The *cho2*-deficient cassette prepared in Example 1-1 was transformed into SCO028 by heat shock and selected on a solid medium containing no uracil. The primary strain thus selected was subjected to a second crossover to prepare a *cho2* gene-deficient strain. The deficient strain thus prepared was designated as "SCO048".

### 4-3. Preparation of SCO028-based opi3-deficient strain ("SCO067")

An *opi3* gene-deficient strain was prepared based on the high fat-producing yeast strain SCO028 prepared in Example 4-1. The *opi3*-deficient cassette prepared in Example 1-2 was transformed into SCO028 by heat shock and selected on a solid medium containing no uracil. The primary strain thus selected was subjected to a second crossover to prepare an *opi3* gene-deficient strain. The deficient strain thus prepared was designated as "SCO067".

### 4-4. Flask test

A flask test of the SCO048 and SCO067 strains prepared in Examples 4-2 and 4-3 was performed.

The two kinds of strains and SCO028 were seeded in a 250 mL corner-baffle flask containing 50 mL of YLMM2 containing no choline or containing 0.3 mM choline at an initial OD of 0.01, and each cultured with shaking at 250 rpm and 30°C for 72 hours. The composition of the YLMM2 is the same as in Example 3-4.

After the culturing was terminated, the culture medium was centrifuged to discard the supernatant, and cells remaining in the lower layer were dried in a dry oven at 60°C overnight, followed by fat extraction. Crude fat extraction was performed according to the feed standard analysis method (feed standard analysis method. 2001. National Livestock Research Institute, Rural Development Administration), and the analyzed OD and the intracellular fat content are shown in Table 8 below. The results in Table 8 are the results of experiments performed in triplicate, and the increase in the fat content was evaluated by mean values thereof.

**[Table 8]**

| Strain | 0D₆₀₀ | | | | Crude fat content [%] | | | |
|---|---|---|---|---|---|---|---|---|
| | Batch 1 | Batch 2 | Batch 3 | Mean | Batch 1 | Batch 2 | Batch 3 | Mean |
| SCO028 | 44.8 | 44.1 | 43.7 | 44.2 | 45.9 | 45.9 | 45.6 | 45.8 |
| SCO028 (w/ CL) | 43.9 | 44.0 | 44.6 | 44.2 | 45.3 | 45.8 | 46.0 | 45.7 |
| SCO048 | 11.2 | 11.4 | 11.3 | 11.3 | 47.2 | 47.1 | 47.6 | 47.3 |
| SCO048 (w/ CL) | 43.3 | 43.4 | 45.0 | 43.9 | 52.3 | 54.4 | 49.9 | 52.2 |
| SCO067 | 11.7 | 11.9 | 11.2 | 11.6 | 47.1 | 47.8 | 46.7 | 47.2 |
| SCO067 (w/ CL) | 44.5 | 44.1 | 44.6 | 44.4 | 50.3 | 50.8 | 50.7 | 50.6 |

As shown in Table 8, the strain in which the *cho2* or *opi3* gene was deleted from the parent strain SCO028 showed an equal or similar fat content in the medium containing no choline, but OD was much reduced.

When 0.3 mM choline was added to the medium, the OD was recovered to the level of the control group, and the effect of increasing the fat content in the strain was also observed, as in Example 3-4. When 0.3 mM choline was added to the medium, SCO048 showed a 14% increase in the fat content, and SCO067 showed a 10% increase in the fat content, as compared with SCO028.

Accordingly, it was also confirmed that when an appropriate amount of choline was added to the strain, in which the *cho2* or *opi3* gene was deleted, the growth of the strain was recovered, and at the same time, the fat content in the strain was increased. In addition, as described in Example 3-4, as the fat content in the strain increased, the increase in the fat content decreased when the *cho2* and *opi3* genes were deleted.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the description preceding them, and therefore all changes and modifications that fall within metes and bounds of the claims or equivalents of such metes and bounds are therefore intended to be embraced by the claims.

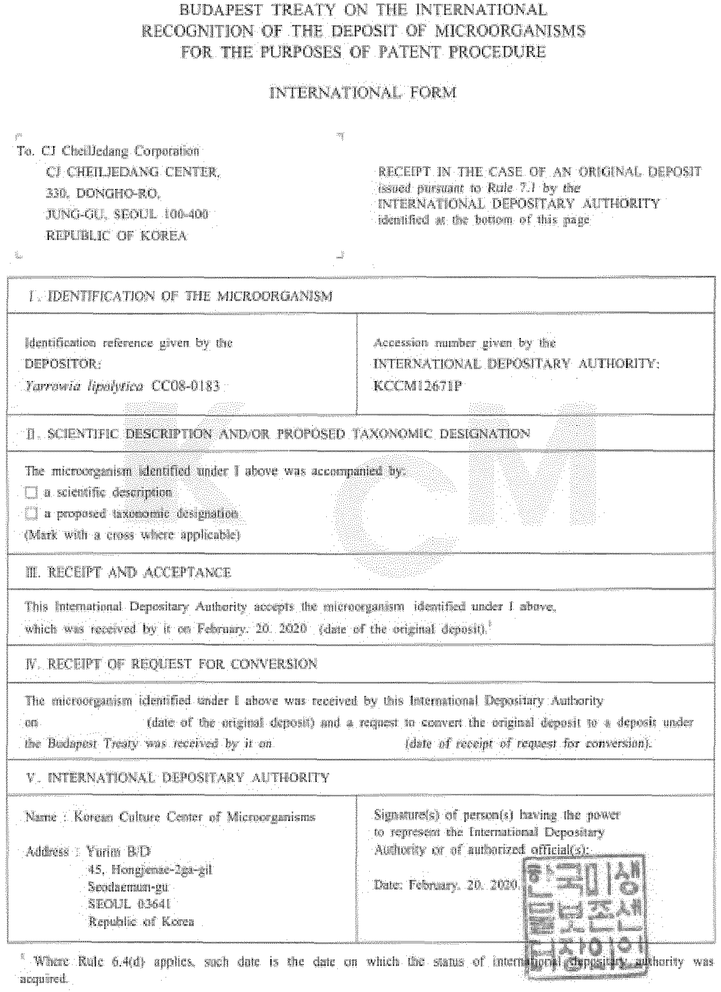
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Yarrowia lipolytica CC08-0183 | Accession number given by the
INTERNATIONAL DEPOSITARY AUTHORITY:
KCCM12671P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on February. 20. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: February. 20. 2020.

¹ Where Rule 6.4(d) applies, such date is the date on which the status of international depositary authority was
acquired.

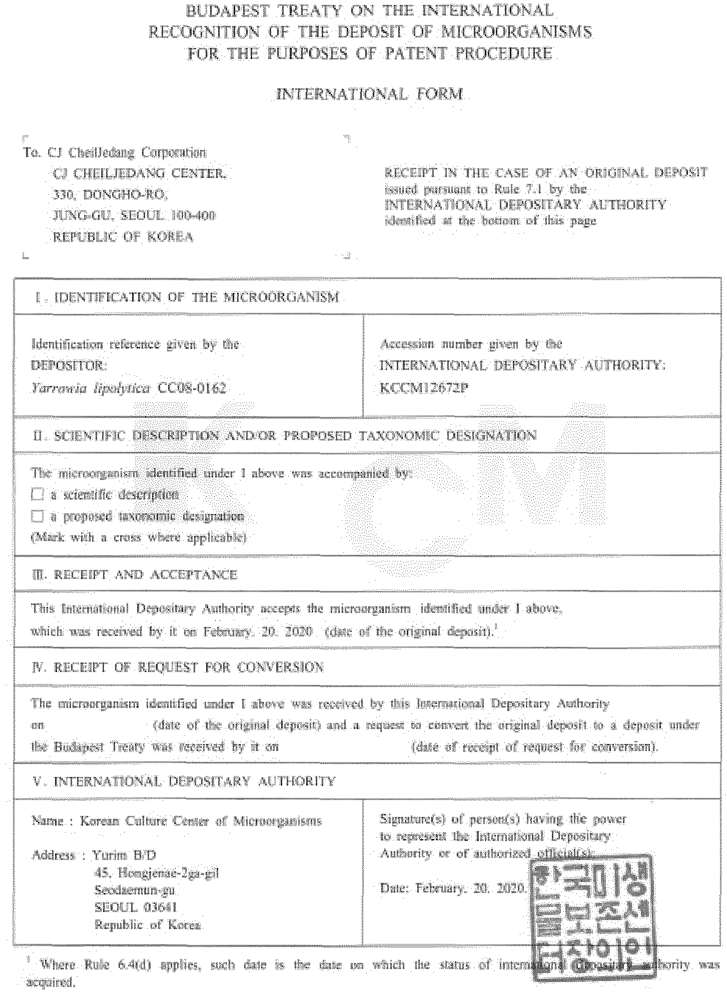
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Yarrowia lipolytica CC08-0162 | Accession number given by the
INTERNATIONAL DEPOSITARY AUTHORITY:
KCCM12672P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on February. 20. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: February. 20. 2020.

¹ Where Rule 6.4(d) applies, such date is the date on which the status of international depositary authority was
acquired.

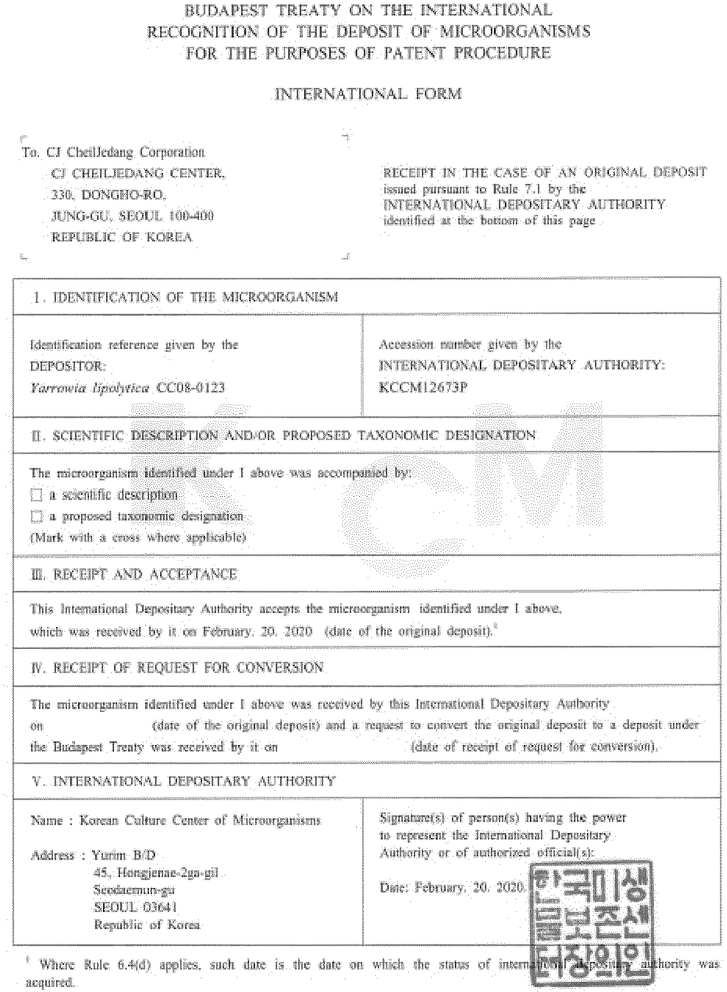
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Yarrowia lipolytica CC08-0123 | Accession number given by the
INTERNATIONAL DEPOSITARY AUTHORITY:
KCCM12673P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on February. 20. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: February. 20. 2020.

¹ Where Rule 6.4(d) applies, such date is the date on which the status of international depositary authority was
acquired.

## Claims

1. A variant strain of the genus *Yarrowia,* wherein activity of phosphatidylethanolamine *N*-methyltransferase (PEMT) or phospholipid methyltransferase is inactivated.

2. The variant strain of the genus *Yarrowia* of claim 1, wherein the phosphatidylethanolamine *N*-methyltransferase is encoded by *cho2* gene, and the *cho2* gene consists of a polynucleotide sequence of SEQ ID NO: 1.

3. The variant strain of the genus *Yarrowia* of claim 2, wherein the *cho2* gene is deleted.

4. The variant strain of the genus *Yarrowia* of claim 1, wherein the phospholipid methyltransferase is encoded by *opi3* gene, and the *opi3* gene consists of a polynucleotide sequence of SEQ ID NO: 11.

5. The variant strain of the genus *Yarrowia* of claim 4, wherein the *opi3* gene is deleted.

6. The variant strain of the genus *Yarrowia* of claim 1, wherein the strain of the genus *Yarrowia* includes *Yarrowia lipolytica.*

7. The variant strain of the genus *Yarrowia* of claim 1, wherein the variant strain of the genus *Yarrowia* is a choline auxotrophic strain.

8. The variant strain of the genus *Yarrowia* of claim 1, wherein the variant strain of the genus *Yarrowia* has an increased fat content, as compared with a wild-type.

9. The variant strain of the genus *Yarrowia* of claim 8, wherein the fat includes triacylglycerol (TAG).

10. A cosmetic composition comprising one or more of the variant strain of the genus *Yarrowia* of any one of claims 1 to 9, a culture of the strain, an extract of the strain, a dry product of the strain, a lysate of the strain, and a fat recovered from one or more of the strain, the culture, the extract, the dry product, and the lysate.

11. A food composition comprising one or more of the variant strain of the genus *Yarrowia* of any one of claims 1 to 9, a culture of the strain, an extract of the strain, a dry product of the strain, a lysate of the strain, and a fat recovered from one or more of the strain, the culture, the extract, the dry product, and the lysate.

12. A feed composition comprising one or more of the variant strain of the genus *Yarrowia* of any one of claims 1 to 9, a culture of the strain, an extract of the strain, a dry product of the strain, a lysate of the strain, and a fat recovered from one or more of the strain, the culture, the extract, the dry product, and the lysate.

13. A medical composition comprising one or more of the variant strain of the genus *Yarrowia* of any one of claims 1 to 9, a culture of the strain, an extract of the strain, a dry product of the strain, a lysate of the strain, and a fat recovered from one or more of the strain, the culture, the extract, the dry product, and the lysate.

14. A method of increasing a fat in a strain, comprising: culturing a variant strain of the genus *Yarrowia,* wherein activity of phosphatidylethanolamine N-methyltransferase (PEMT) or phospholipid methyltransferase is inactivated.

15. The method of increasing a fat in a strain of claim 14, wherein the culturing the strain is culturing the strain in a medium containing choline.

16. The method of increasing a fat in a strain of claim 15, wherein a concentration of the choline in the medium is 0.05 mM to 5 mM.

17. The method of increasing a fat in a strain of claim 15, wherein a C/N ratio in the medium is 40 to 80.

18. The method of increasing a fat in a strain of claim 14, wherein the fat includes triacylglycerol (TAG).

19. A method of preparing a fat, comprising: culturing a variant strain of the genus *Yarrowia,* wherein activity of phosphatidylethanolamine *N-*methyltransferase (PEMT) or phospholipid methyltransferase is inactivated.

20. The method of preparing a fat of claim 19, further comprising: recovering the fat from the strain, a culture thereof, an extract thereof, a dry product thereof, or a lysate thereof, after the culturing the strain.

21. The method of preparing a fat of claim 19, wherein the culturing the strain is culturing the strain in a medium containing choline.

22. The method of preparing a fat of claim 21, wherein a C/N ratio in the medium is 40 to 80.

23. Use of a variant strain of the genus *Yarrowia,* wherein activity of phosphatidylethanolamine *N*-methyltransferase (PEMT) or phospholipid methyltransferase is inactivated; a culture of the strain; an extract of the strain; a dry product of the strain; a lysate of the strain; a cosmetic composition, a food composition, a feed composition, or a medical composition, each including one or more of the strain, the culture, the extract, the dry product, and the lysate, and a fat recovered from one or more of the strain, the culture, the extract, the dry product, and the lysate in fat production.
